# EUROPEAN PATENT APPLICATION

(11) **EP 4 606 341 A1**
(43) Date of publication of application: **27.08.2025**
(21) Application number: 23878784.0
(22) Date of filing: 10.08.2023
(51) Int. Cl.: A61B 34/35, A61B 17/00

(54) **SURGICAL ROBOT, AND DETECTION MECHANISM AND DETECTION METHOD FOR SURGICAL ROBOT**

(30) Priority: 21.10.2022 CN 202211292645; 21.10.2022 CN 202222779464 U
(71) Applicant: Agibot Medtech (Suzhou) Co., Ltd., Suzhou, Jiangsu 215123 (CN)
(72) Inventor: WANG, Yupu, Suzhou, Jiangsu 215123 (CN); PENG, Cheng, Suzhou, Jiangsu 215123 (CN); XU, Min, Suzhou, Jiangsu 215123 (CN)
(74) Representative: Tranvouez, Edern Morgan
(86) International application number: PCT/CN2023/112188
(87) International publication number: WO 2024/082788

(57) **Abstract**

A surgical robot, and a detection mechanism and detection method for the surgical robot. The surgical robot includes: an actuation box (10) capable of carrying a power device that provides power, and includes an actuation box top plate (2) arranged at a top portion, the actuation box top plate (2) defining a central axis (Y); a sterile isolation plate (20) detachably coupled to an upper side of the actuation box top plate (2); an instrument box (30) including an instrument box bottom plate (5) arranged at a bottom portion, and the instrument box bottom plate (5) is detachably coupled to an upper side of the sterile isolation plate (20); and a coupling monitoring mechanism, which is arranged on the actuation box top plate (2) and includes at least one pair of first monitoring switches (61) and at least one pair of second monitoring switches (62), wherein the at least one pair of first monitoring switches (61) are exposed to outside relative to the actuation box top plate (2), and the at least one pair of first monitoring switches (61) are arranged away from each other at two sides of the central axis (Y), the at least one pair of second monitoring switches (62) are exposed to outside relative to the actuation box top plate (2), and the at least one pair of second monitoring switches (62) are arranged away from each other at two sides of the central axis (Y).

## Description

This application claims priority to Chinese patent application No. 202211292645.8 filed on October 21, 2022, and priority to Chinese patent application No. 202222779464.X filed on October 21, 2022, the entire disclosures of which are incorporated by reference into the present disclosure.

### FIELD OF THE INVENTION

The present disclosure relates to the field of medical instruments, and in particular to a surgical robot, and a detection mechanism and a detection method for the surgical robot.

### BACKGROUND OF THE INVENTION

The surgical robot system has been a research hotspot in the field of medical instruments in recent years. It can assist in minimally invasive surgery and has been widely accepted by the market due to its advantages such as small surgical wounds, low bleeding, precise surgical operations, and rapid patient recovery. The surgical robot system includes a main control terminal for a doctor to issue instructions, multiple robotic arms equipped with actuation boxes at the slave end, and an instrument assembly mounted on the robotic arms. The doctor controls movements of the robotic arms and the instrument assembly through the main control terminal to complete various operations such as resecting and suturing in surgical positions.

The instrument assembly includes a sterile isolation plate detachably coupled to the upper side of the actuation box, and an instrument box detachably coupled to the sterile isolation plate. The actuation box is equipped therein with a power device for providing power, and the surgical instrument is provided in the instrument box and is in transmission connection with the power device to perform various surgical actions under the drive of the power device.

However, it is difficult to determine whether the actuation box and the sterile isolation plate, and the sterile isolation plate and the instrument box are securely coupled.

### SUMMARY OF THE INVENTION

It is provided according to embodiments of the present disclosure a surgical robot, a detection mechanism and a detection method for the surgical robot.

According to some embodiments of the present disclosure, a surgical robot is provided, which includes:
An actuation box which can carry a power device that provides power. The actuation box includes an actuation box top plate arranged at a top portion, and the actuation box top plate defines a central axis;
A sterile isolation plate that is detachably coupled on the actuation box top plate;
An instrument box, the instrument box includes an instrument box bottom plate arranged at a bottom portion, and the instrument box bottom plate is detachably coupled on the sterile isolation plate; and
A coupling monitoring mechanism that is arranged on the actuation box top plate, the coupling monitoring mechanism includes at least one pair of first monitoring switches and at least one pair of second monitoring switches, wherein the at least one pair of first monitoring switches are exposed to the outside relative to the actuation box top plate, and are arranged at both sides of the central axis away from each other; the at least one pair of second monitoring switches are exposed to the outside relative to the actuation box top plate, and are arranged at both sides of the central axis away from each other; and
The first monitoring switch is configured to send out a first trigger signal when the sterile isolation plate is coupled to the actuation box top plate, and the second monitoring switch is configured to send out a second trigger signal when the instrument box bottom plate is coupled to the sterile isolation plate.

According to some embodiments of the present disclosure, a detection mechanism for a surgical robot is provided, wherein the surgical robot includes an actuation box having a actuation box top plate, a sterile isolation plate and an instrument box having an instrument box bottom plate, and wherein the instrument box is detachably coupled to the actuation box through the sterile isolation plate; the detection mechanism includes a plurality of pressing devices arranged on the actuation box top plate and/or the instrument box bottom plate, and the pressing device includes a through-beam switch, an axially movable pressing member and a reset spring, and wherein the through-beam switch includes a light transmitter and a light receiver arranged opposite to each other, and the pressing member has a first position and a second position;
In the first position, at least a portion of the pressing member is protruded axially relative to the actuation box or the instrument box and is separated from the through-beam switch; and
In the second position, the pressing member is completely received in the actuation box or the instrument box, and at least a portion of the pressing member is located between the light transmitter and the light receiver; and the reset spring abuts against the pressing member and provides a force for the pressing member towards the first position.

According to some embodiments of the present disclosure, a detection method for a surgical robot is provided, which includes the following steps:
S1: collecting a first trigger signal, and generating a first coupling success information indicating that the sterile isolation plate and the actuation box are successfully coupled, if each first monitoring switch sends the first trigger signal, and sending a ready-to-connect information to the outside to be ready to couple the instrument box; otherwise, generating a first coupling failure information and sending a first fault information to the outside;
S2: collecting a second trigger signal, and generating a second coupling success information indicating that the instrument box and the sterile isolation board are successfully coupled, if each second monitoring switch sends the second trigger signal; otherwise, sending a second coupling failure information to the outside.

According to some embodiments of the present disclosure, a detection method for a surgical robot is provided, which includes:
Collecting a first trigger signal, and confirming that the sterile isolation plate is successfully coupled to the actuation box in response to first trigger signals sent by each of the at least one pair of first monitoring switches;
Sending a ready-to-couple information to be ready to couple the instrument box;
Collecting a second trigger signal, and confirming that the instrument box and the sterile isolation board are successfully coupled in response to second trigger signals sent by each of the at least one pair of second monitoring switches.

### BRIEF DESCRIPTION OF THE DRAWINGS

FIG. 1 is a schematic diagram of an application scenario of a surgical robot provided according to some embodiments of the present disclosure;
FIG. 2 is a schematic exploded structural diagram of an actuation box top plate, a sterile isolation plate, and an instrument box bottom plate of a surgical robot provided according to some embodiments of the present disclosure;
FIG. 3 is another schematic exploded structural diagram of an actuation box top plate, a sterile isolation plate, and an instrument box bottom plate of a surgical robot provided according to some embodiments of the present disclosure;
FIG. 4 is a side view of an actuation box top plate, a sterile isolation plate, and an instrument box bottom plate of a surgical robot provided according to some embodiments of the present disclosure;
FIG. 5 is a sectional view of an actuation box top plate of a surgical robot provided according to some embodiments of the present disclosure;
FIG. 6 is a sectional view of an actuation box top plate and a sterile isolation plate of a surgical robot provided according to some embodiments of the present disclosure;
FIG. 7 is a sectional view of an actuation box top plate, a sterile isolation plate, and an instrument box bottom plate of a surgical robot provided according to some embodiments of the present disclosure;
FIG. 8 is a top view of an actuation box top plate of a surgical robot provided according to some embodiments of the present disclosure;
FIG. 9 is a sectional view along line A-A in FIG. 8;
FIG. 10 is a logic judgment diagram of a coupling monitoring mechanism of a surgical robot provided according to some embodiments of the present disclosure;
FIG. 11 is a schematic flow diagram of a detection method for an actuation box, a sterile isolation plate, and an instrument box of a surgical robot provided according to some embodiments of the present disclosure;
FIG. 12 is an exploded schematic diagram of an actuation box top plate, a sterile isolation plate, and an instrument box bottom plate of a surgical robot provided according to some embodiments of the present disclosure;
FIG. 13 is a partial top view of an actuation box top plate of a surgical robot provided according to some embodiments of the present disclosure;
FIG. 14 is a sectional view along line B-B in FIG. 3;
FIG. 15 is another sectional view along line B-B in FIG. 3;
FIG. 16 is a schematic structural diagram of a coupling monitoring mechanism of a surgical robot provided in some embodiments of the present disclosure; and
FIG. 17 is another schematic structural diagram of the coupling monitoring mechanism of the surgical robot provided in some embodiments of the present disclosure.

Reference signs in the figures:
10. actuation box; 20. sterile isolation board; 30. instrument box; 40. robotic arm; 50. surgical instrument;
1. actuation box body;
2. actuation box top plate; 21. power output member; 22. first signal reader; 23. first signal transmission device; 24. first coupling groove;
31. power transmission member; 32. signal through hole; 33. second coupling member; 34. downward pressing through hole; 35. transmission rod;
4. instrument box body;
5. instrument box bottom plate; 51. power through hole; 52. instrument hole; 53. second signal reader; 54. second signal transmission device; 55. second coupling groove;
61. first monitoring switch; 62. second monitoring switch; 63. Hall sensor; 64. permanent magnet; 65. circuit board;
Y. central axis;
11. groove; 12. second through hole; 13. third through hole;
3. PCB ;
41. light transmitter; 42. light receiver;
6. reset spring; 7. transmission rod;
8. pressing member; 81. main shaft portion; 82. brim portion; 83. pressing boss.

### DETAILED DESCRIPTION OF THE EMBODIMENTS

In order to explain the technical contents, structural features, objectives and effects of the present disclosure in detail, technical solutions of embodiments of the present disclosure will be described below in conjunction with the accompanying drawings in the embodiments of the present disclosure.

In the present disclosure, spatially relative terms such as "beneath", "below", "under", "lower", "on", "upper", "above", "higher", "side" (e.g., in the "side wall"), etc., are used to describe relationship of one element to another (other) element as shown in the accompanying drawings. The spatially relative terms are intended to include different orientations of the device in use, in operation, and/or in manufacturing in addition to those depicted in the accompanying drawings. For example, if the device shown in the accompanying drawing is turned over, the elements described as "below" or "beneath" other elements or features will then be positioned as "on" the other elements or features. Therefore, the exemplary term "below" may include both on and below orientations. In addition, the device can be positioned otherwise (e.g., rotated 90 degrees or at other orientations), so the spatially relative terms used herein are interpreted accordingly.

In the present disclosure, the term "coupled to" means that two components are directly or indirectly fixed relative to each other through a coupling mechanism. For example, the expression "the instrument box bottom plate is coupled to the upper side of the sterile isolation plate" should be understood as the instrument box bottom plate may be directly connected to the sterile isolation plate through a coupling mechanism, or be directly coupled to the actuation box top plate through a coupling mechanism and is maintained fixed relative to the sterile isolation plate, unless otherwise specified.

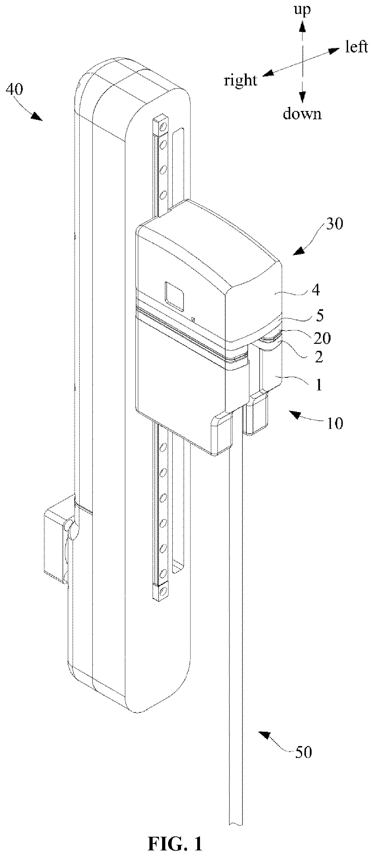

The surgical robot includes a main control terminal configured for the operator to issue instructions, multiple robotic arms arranged at the slave terminal, and an instrument assembly (such as the actuation box, the sterile isolation board and the instrument box shown in FIG. 1) mounted on the robotic arms. The instrument assembly may carry various surgical instruments 50 (which may be a clamp, a gripper, a stapler, a cautery tool, a linear cutter, etc.) for surgery. The instrument assembly is configured to be in communication connection with the main control terminal signal at the far end such that the operator remotely control the surgical instrument 50 and perform various surgical operations (such as cutting, suturing, hemostasis, etc.).

In some examples, as shown in FIG. 1, the surgical robot provided according to some embodiments of the present disclosure includes an actuation box 10 that belongs to a robotic arm and can carry a power device (not shown in the figure), a sterile isolation plate 20 that belongs to an instrument assembly and can be detachably coupled to an upper side of the actuation box 10, an instrument box 30 that belongs to the instrument assembly and can be detachably coupled to an upper side of the sterile isolation plate 20, and a coupling monitoring mechanism.

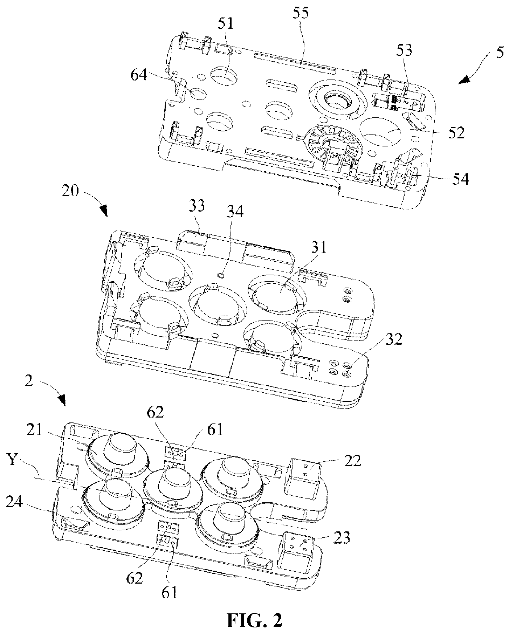

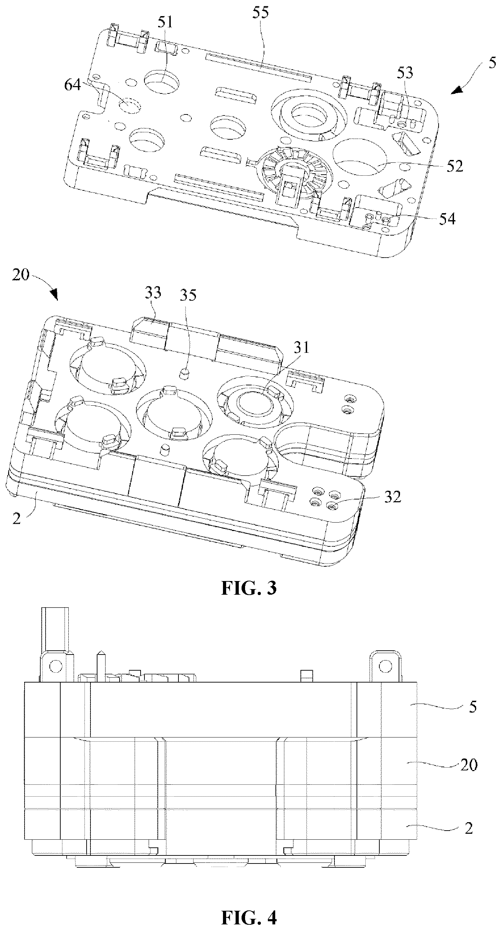

The actuation box 10 may be driven by the robotic arm 40 to move up and down. In conjunction with FIG. 2 and FIG. 3, in some embodiments of the present disclosure, the actuation box 10 includes an actuation box body 1 and an actuation box top plate 2 detachably mounted on the actuation box body 1. The actuation box body 1 defines a power cavity (not shown in the figure) with an upper opening, and a power device (not shown in the figure) that can output power to the surgical instrument 50 is arranged in the power cavity.

As shown in FIG. 2, a plurality of power output members 21 are arranged on the actuation box top plate 2, and the power output members 21 are movably arranged on the actuation box top plate 2; a first signal reader 22 and a first signal transmission device 23 are fixedly arranged on the actuation box top plate 2. The power output members 21 are all protruded upward and are transmission-connected with the power device in the power cavity to output power to the outside while isolating the power device; the first signal reader 22 is configured to read and rewrite the information of the surgical instrument; the first signal transmission device 23 is configured to transmit part of the electrical signal in the surgical instrument.

The instrument box 30 includes an instrument box body 4 and an instrument box bottom plate 5, and the instrument box bottom plate 5 is detachably mounted on the bottom of the instrument box body 4. An instrument cavity is defined in the instrument box body 4 and is exposed from the bottom, and transmission mechanisms corresponding to various surgical instruments 50 may be arranged in the instrument cavity.

The instrument box bottom plate 5 is provided thereon with a plurality of power through holes 51 and one instrument hole 52; a second signal reader 53 and a second signal transmission device 54 are fixedly disposed on the instrument box bottom plate 5. The power through hole 51 is configured for mechanical coupling between the transmission mechanisms in the instrument cavity and a power transmission member 31, so as to guide the power to the surgical instrument 50; the instrument hole 52 is configured for a rod of the surgical instrument 50 to pass through the instrument box 30. The second signal reader 53 is configured to control the surgical instrument 50 to act accordingly in response to remote control signal from the main control terminal; and the second signal transmission device 54 is configured to transmit part of the electrical signal in the instrument assembly.

The sterile isolation board 20 is configured to isolate the actuation box 10 from the instrument box 30 to prevent the actuation box 10 from contaminating the instrument box 30 and the surgical instruments 50 carried on the instrument box 30. The sterile isolation board 20 is disposed thereon with the power transmission members 31 (see FIG. 2), the number of which is consistent with the number of the power output members 21. The power transmission members 31 are movably arranged on the sterile isolation board 20. In some examples, the power transmission members 31 are in contact with the power output members 21 to transmit power from the actuation box 10 to the outside. In some examples, the sterile isolation board 20 is also provided thereon with a plurality of signal through holes 32, and the signal through holes 32 are configured for signal transmission to realize signal connection between the first signal reader and the second signal reader, and signal connection between the first signal transmission device and the second signal transmission device.

A first coupling mechanism is disposed between the actuation box top plate 2 and the sterile isolation plate 20 to realize the coupling of the two. The first coupling mechanism includes multiple first coupling members (not shown in the figure) and multiple first coupling grooves 24. The first coupling members are disposed on the bottom of the sterile isolation plate 20, and the first coupling grooves 24 are disposed on the actuation box top plate 2. The first coupling grooves 24 are configured to match or fit with the first coupling members. In some examples, the first coupling members are configured to act synchronously to complete the coupling and unlocking actions.

In some examples, a second coupling mechanism is arranged between the sterile isolation plate 20 and the instrument box 30 to realize the coupling of the two. The second coupling mechanism includes a pair of second coupling members 33 and a pair of second coupling grooves 35, wherein the second coupling members 33 are located on the top portion of the sterile isolation plate 20, and the pair of second coupling grooves 35 are disposed on the instrument box bottom plate 5. The second coupling grooves 55 are configured to match or fit with the second coupling members 33. In some examples, the pair of second coupling members 33 are configured to act synchronously to complete the coupling and unlocking actions.

It should be noted that the above mentioned first coupling mechanism and second coupling mechanism are non-limiting examples for illustration only. On the premise that the actuation box, the sterile isolation plate and the instrument box can be relatively fixed via coupling, their specific coupling manners and configurations do not limit the protection scope of the present disclosure.

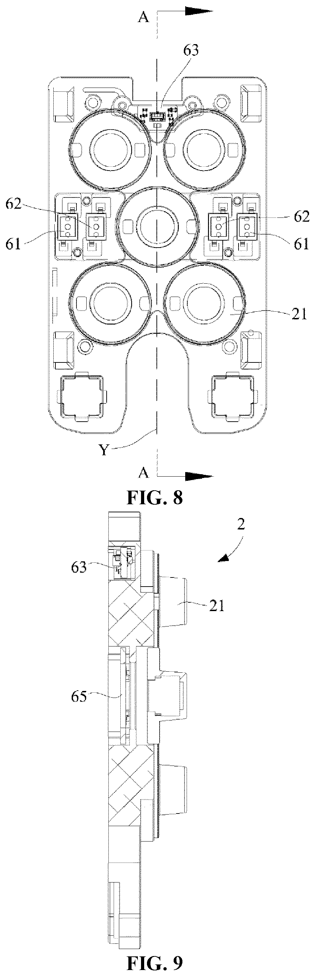

Referring to FIG. 2 to FIG. 3, the coupling monitoring mechanism is configured to monitor coupling status between the actuation box 10, the sterile isolation plate 20 and the instrument box 30. The coupling monitoring mechanism includes a pair of first monitoring switches 61, a pair of second monitoring switches 62 and a Hall sensor 63 (see FIG. 8).

The pair of first monitoring switches 61 are configured to monitor whether the sterile isolation plate 20 is coupled to the actuation box 10. The pair of second monitoring switches 62 are configured to monitor whether the instrument box 30 is coupled to the sterile isolation plate 20. The Hall sensor 63 is configured to monitor whether the instrument box 30 is placed above the actuation box 10.

As shown in FIG. 2, the actuation box top plate 2 defines a central axis Y extending forward and backward. In some examples, the first monitoring switch 61 is a mechanical trigger switch, and is configured to be triggered when being applied with pressure and send out a first trigger signal. The pair of first monitoring switches 61 are fixedly arranged on the actuation box top plate 2 away from each other. In some examples, the pair of first monitoring switches 61 are arranged at both sides of the central axis Y in left-right mirror images manner. With this arrangement manner, the distance between the pair of first monitoring switches 61 may be increased as much as possible, avoiding leaving out one corner of the sterile isolation plate 20 and the actuation box top plate 2 that is not firmly coupled, which may otherwise causing warped coupling. It can be understood that, only when each of the pair of first monitoring switches 61 sends out the first trigger signal, can it be considered that the sterile isolation plate 20 and the actuation box top plate 2 are successfully coupled.

In other words, in some examples of this disclosure, there are at least several states between the sterile isolation plate 20 and the actuation box top plate 2:

### A first state

The sterile isolation plate 20 is not installed on the actuation box top plate 2. At this time, neither of the pair of first monitoring switches 61 is triggered, that is, neither of the pair of first monitoring switches 61 sends a first trigger signal; or, the controller does not receive a first trigger signal sent by any of the pair of first monitoring switches 61.

### A second state

The sterile isolation plate 20 is installed to the actuation box top plate 2, and the controller receives a first trigger signal from one of the pair of first monitoring switches 61, but fails to receive a first trigger signal from the other first monitoring switch 61. In this case, the sterile isolation plate 20 and the actuation box top plate 2 are not firmly coupled, there may be warped coupling, and it needs to couple the sterile isolation plate 20 to the actuation box top plate 2 again, or to couple one side of the sterile isolation plate 20 where the first monitoring switch 61 that does not send the first trigger signal is located again.

### A third state

The sterile isolation plate 20 is mounted on the actuation box top plate 2, and the controller receives first trigger signals from the pair of first monitoring switches 61. At this time, the sterile isolation plate 20 is firmly coupled to the actuation box top plate 2. In this way, it is ensured that the sterile isolation plate 20 and the actuation box top plate 2 are firmly coupled.

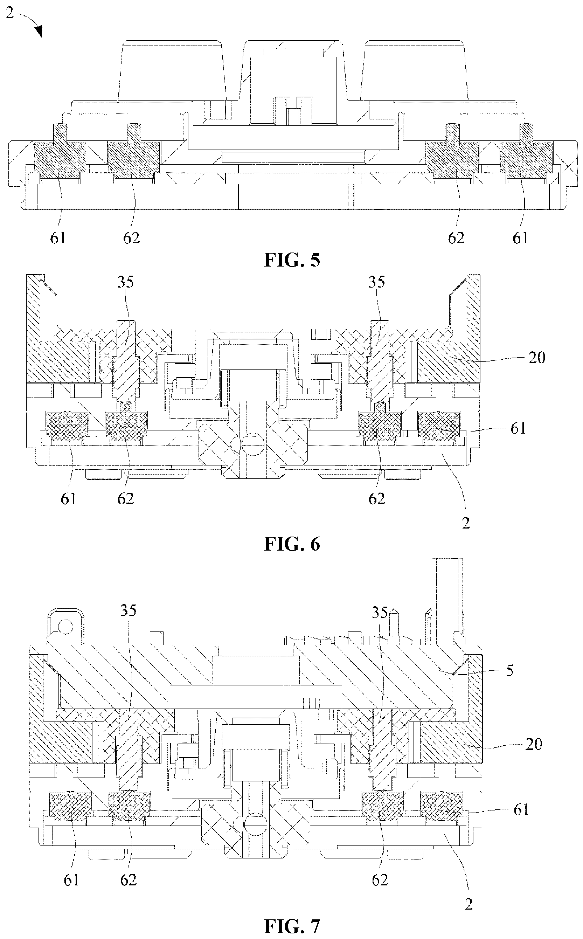

In some examples, referring to FIG. 4 to FIG. 6, the first monitoring switch 61 includes a first pressing rod (not shown in the figure) and a first trigger switch (not shown in the figure). The first pressing rod protrudes upward and is retractable. The first trigger switch is configured to cooperate with the first pressing rod. The first pressing rod is configured to maintain a state of protruding outward in a natural state (which may be achieved by providing a corresponding elastic member or magnetic member). The first trigger switch is configured to be triggered when the first pressing rod is compressed and send out a first trigger signal. The type of the first trigger switch is not limited to a magnetic switch, a mechanical trigger switch or a through-beam switch. In some examples, there may be other numbers of the first monitoring switches and/or the first monitoring switches may be positioned otherwise, based on actual requirements, for example, the number of the first monitoring switches may be set to be consistent with the number of the first coupling grooves, and the first monitoring switches are respectively located at corresponding first coupling grooves. Under the premise that all the first monitoring switches can be triggered when the sterile isolation plate is properly coupled to the actuation box top plate, the specific number and positions of the first monitoring switches do not limit the protection scope of the present disclosure.

In some examples, referring to FIGS. 4, 6 and 7, a pair of second monitoring switches 62 are fixedly arranged on the actuation box top plate 2 away from each other. In some examples, the pair of second monitoring switches 62 are arranged at both sides of the central axis Y in a left-right mirror images manner. The second monitoring switch 62 includes a second pressing rod (not shown in the figure) and a second trigger switch (not shown in the figure), wherein the second pressing rod protrudes upward and is retractable; the second trigger switch is configured to cooperate with the second pressing rod. In some examples, the second pressing rod is configured to maintain a state of protruding outward in a natural state. The second trigger switch is configured to be triggered when the second pressing rod is compressed, and to send a second trigger signal outward. Among them, the type of the second trigger switch is not limited to a magnetic switch, a mechanical trigger switch or through-beam switch.

In some examples, as shown in FIG. 2, the sterile isolation plate 20 is provided thereon with a downward pressing through hole 34 aligned with the second monitoring switch 62. The number of the downward pressing through holes 34 matches the number of the second monitoring switches 62. A transmission rod 35 is disposed on the sterile isolation plate 20, and the transmission rod 35 is slidably matched with the downward pressing through hole 34. When the sterile isolation plate 20 is coupled on the actuation box top plate 2, a lower end of the transmission rod 35 contacts the second pressing rod of the second monitoring switch 62 to transmit a pressure from the instrument box bottom plate 5 to the second pressing rod.

Referring to FIG. 2 and FIG. 8, the Hall sensor 63 is fixedly arranged on the actuation box top plate 2. In some examples, the Hall sensor 63 is located on the central axis Y. A permanent magnet 64 is fixedly arranged on the instrument box bottom plate 5, and the permanent magnet 64 cooperates with the Hall sensor 63. When the actuation box 10, the sterile isolation plate 20 and the instrument box 30 are sequentially coupled, the permanent magnet 64 is located directly above the Hall sensor 63. With the Hall sensor 63 sensing a distance of the permanent magnet 64, it can be judged whether the instrument box 30 is placed or coupled on the actuation box 10, or it may provide basis for the next action of the actuation box 10 (e.g., providing basis for judging the distance between the actuation box 10 and the instrument box 30 when replacing the surgical instrument 50 to avoid collision between the two).

In some examples, the permanent magnet 64 may be attached to the instrument box bottom plate 5 by gluing, hot riveting, injection mosaic, etc. It can be understood that in order to shorten the distance between the Hall sensor 63 and the permanent magnet 64, a lower end surface of the permanent magnet 64 may be as close as possible to a lower surface of the instrument box bottom plate 5. In some examples, the Hall sensor is arranged on the instrument box bottom plate, and the permanent magnet may be arranged on the actuation box top plate; alternatively, the Hall sensor may be arranged on the actuation box top plate, and the permanent magnet is arranged on the instrument box bottom plate.

In some embodiments of the present disclosure, the coupling state of the instrument box bottom plate and the sterile adapter 20 may be determined according to the second trigger signal sent by the second monitoring switch 62. The coupling state of the instrument box bottom plate and the sterile adapter 20 may also have three states, which are the same or similar to the three states of the sterile adapter 20 and the actuation box top plate mentioned in the previous embodiments of the present disclosure. Reference may be made to the detailed description of the previous embodiments of the present disclosure, and the present disclosure will not repeat them.

It is understood that the Hall sensor 63 is configured to confirm whether the instrument box 30 is located above the actuation box 10. In some examples, strokes of the first and second pressing rods are small, and the coupling states between the actuation box 10, the sterile isolation plate 20 and the instrument box 30 can be accurately determined.

In conjunction with FIG. 9, in some examples, the surgical instrument provided according to the embodiments of the present disclosure further includes: a circuit board 65, and the first monitoring switch and the second monitoring switch are both disposed on the circuit board 65. In some examples, the Hall sensor 63 may be disposed on the circuit board 65, or may be arranged separately, depending on specific space size and the size of the circuit board, and other factors.

In some examples, the actuation box top plate 2 has a groove (not shown in the figure) exposed from the bottom portion, and the circuit board 65 and the Hall sensor 63 are fixedly arranged in the groove, which can shorten the distance between the Hall sensor 63 and the permanent magnet 64, and the Hall sensor 63, the first monitoring switch and the second monitoring switch can be protected from being collided, thereby extending the service life of the surgical robot.

In some other examples, a metal protective layer (not shown in the figure) is also arranged on the actuation box top plate 2 above the circuit board 65.

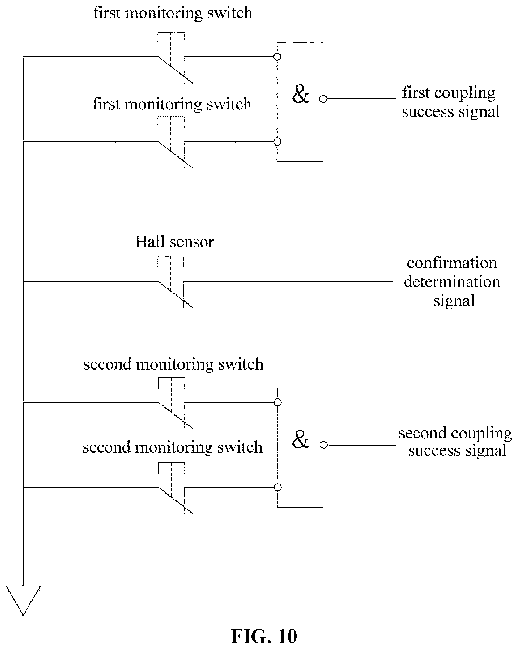

FIG. 10 is a logic judgment diagram of the coupling monitoring mechanism of the surgical robot provided in the present disclosure.

As shown in FIG. 10, in some examples, the pair of first monitoring switches 61 are connected in parallel. When the pair of monitoring switches 61 are both triggered and send out the first trigger signals, the circuit board 65 generates a first coupling success signal indicating that the sterile isolation plate 20 is properly coupled to the actuation box 10; otherwise (for example, any one of the pair of first monitoring switches 61 does not send out the first trigger signal, or neither of the pair of first monitoring switches 61 sends out the first trigger signal), a first coupling failure signal is generated.

In some examples, the pair of second monitoring switches 62 are connected in parallel. When the second pair of monitoring switches 63 are both triggered and send out second trigger signals, the circuit board 65 generates a second coupling success signal indicating that the instrument box 30 is properly coupled to the sterile isolation plate 20; otherwise (for example, any one of the pair of second monitoring switches 62 does not send out the second trigger signal, or neither of the pair of second monitoring switches 62 sends out the second trigger signal), a second coupling failure signal is generated.

In some examples, the Hall sensor 63 selectively generates a confirmation signal indicating that the instrument box 30 is placed on the actuation box 10 based on a distance of the permanent magnet 64. It can be understood that the coupling monitoring mechanism of the surgical robot provided by the present disclosure adopts a partially redundant design and has a certain fault detection function. For example, when the circuit board 65 generates a third judgment signal but not the second judgment signal, it means that at least one of the Hall sensor 63 and the second monitoring switch 62 is faulty.

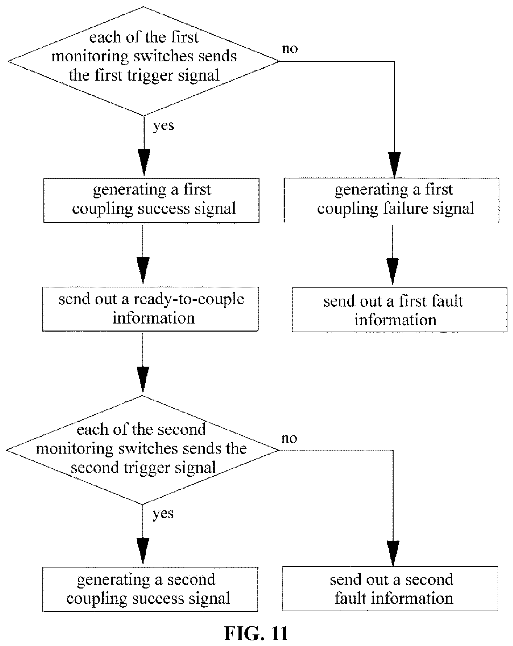

In conjunction with FIG. 11, the present disclosure further provides a detection method for a surgical robot, including the following steps:
S1: collecting a first trigger signal, and, in a case that the pair of first monitoring switches both send out first trigger signals, generating a first coupling success information indicating that the sterile isolation plate and the actuation box are successfully coupled. In other words, in response to both of the pair of first monitoring switches send out the first trigger signals, it is confirmed that the sterile isolation plate and the actuation box are successfully coupled.

Otherwise, a first coupling failure information is generated. That is, in response to any one of the pair of first monitoring switches does not send the first trigger signal, it is confirmed that the sterile isolation plate and the actuation box have failed to be coupled, and the first coupling failure information is generated.

S2: after generating the first coupling success information, sending a ready-to-couple information to the outside to prepare for coupling the instrument box; or sending a first fault information to the outside after receiving the first coupling failure information;
Wherein, the first fault information includes at least one of the followings: the indicator light of the actuation box flashes or changes color, a voice prompt indicating that the isolation board is incorrectly coupled, and a prompt shown on a doctor end-display indicating that the sterile isolation board is incorrectly coupled.

S3: collecting a second trigger signal, then generating a second coupling success information indicating that the instrument box and the sterile isolation board are successfully coupled if each of the second monitoring switches sends the second trigger signal. In other words, in response to both of the pair of second monitoring switches send the second trigger signal, it is confirmed that the instrument box and the sterile isolation board are successfully coupled.

Otherwise, a second coupling failure information is generated. That is, in response to any one of the pair of second monitoring switches does not send the second trigger signal, it is confirmed that the coupling between the instrument box and the sterile isolation board fails, and a second fault information is sent. In some examples, the second fault message may also be understood as a second coupling failure information.

The second fault information includes at least one of the followings: the indicator light of the actuation box flashes or changes color, a voice prompt indicating that the instrument box is incorrectly coupled, and a prompt shown on a doctor end-display indicating that the instrument box is incorrectly coupled.

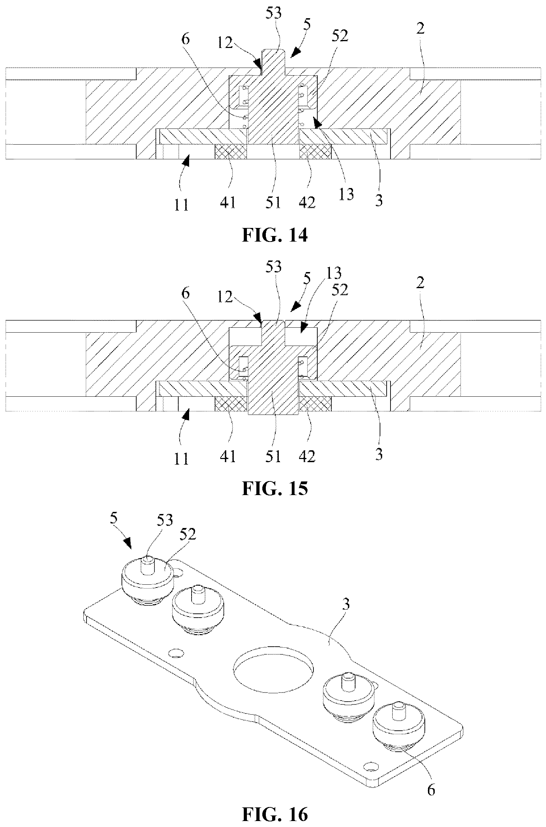

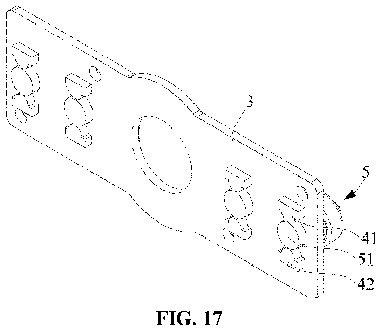

The actuation box top plate 2 and the instrument box bottom plate 5 are based on the detail description of the aforementioned embodiments of the present disclosure. According to other embodiments of the present disclosure, as shown in FIG. 16 and FIG. 17, the coupling monitoring mechanism (which may also be called as a detection mechanism in some examples) includes a printed circuit board (Printed Circuit Board, referred to as PCB in brief) 3 and several pressing devices. The pressing devices are mounted on the PCB 3. A first through hole (not shown in the figure) is provided on the PCB 3, and the number of the first through holes is consistent with the number of the pressing devices, and each first through hole corresponds to one pressing device.

In some examples, the pressing device includes a through-beam switch, a pressing member 8 and a reset spring 6; wherein the through-beam switch is fixedly disposed at the first through hole, and the pressing member 8 fits with the first through hole in a shaft-hole manner.

That is to say, in some other examples of the present disclosure, the through-beam switch is taken as an example for the first monitoring switch 61 and the second monitoring switch 62 for exemplary illustration. It can be understood that in some examples, the coupling monitoring mechanism may also be called as a detection mechanism, and the detection mechanism is configured to monitor coupling states between the actuation box top plate 2, the sterile isolation plate 20 and the instrument box bottom plate 5. In the embodiment of the present disclosure, the coupling states between the actuation box top plate 2, the sterile isolation plate 20 and the instrument box bottom plate 5 may refer to the detailed description of the aforementioned embodiments of the present disclosure, and thus will not be repeated herein.

The through-beam switch include a light transmitter 41 and a light receiver 43. The light transmitter 41 and the light receiver 42 are disposed opposite to each other at two sides of the first through hole. Both the light transmitter 41 and the light receiver 42 are electrically connected to the PCB 3.

In some examples, the light receiver 42 is a photoresistor. It can be understood that when the light receiver 42 receives light from the light transmitter 41, the resistance of the light receiver 42 decreases, which may be regarded as a conducting state in the circuit, the through-beam switch is in an activated state, and the PCB 3 outputs a first electrical signal; and when the light between the light transmitter 41 and the light receiver 42 is blocked, the resistance of the light receiver 42 increases, which may be regarded as an open circuit state in the circuit, the through-beam switch is in a blocked state, and the PCB 3 outputs a second electrical signal. Therefore, it can be determined whether the through-beam switch is in an activated state by identifying the signal sent by the PCB.

The pressing member 8 includes a main shaft portion 81, a brim portion 51 and a pressing boss 83. Wherein, the main shaft portion 81 fits with the first through hole in a shaft-hole manner; the brim portion 51 extends axially along an outer edge of the main shaft portion 81, and the pressing boss 83 is located on an upper side of the main shaft portion 81.

The brim portion 82, the main shaft portion 81 and the pressing boss 83 are integrally formed and their diameters decrease successively. The main body of the pressing member 8 and the through-beam switchs are located at two opposite sides of the PCB 3. The pressing member 8 is configured to be movable axially relative to the first through hole and has a first position and a second position. In the first position, the pressing member 8 is away from the through-beam switch, while in the second position, at least part of the main shaft portion 81 of the pressing member 8 extends between the light transmitter 41 and the light receiver 42.

It can be understood that when the pressing member 8 is located at the first position, the through-beam switch is in an activated state, and the PCB 3 sends a first signal to the outside; when the pressing member 8 is located at the second position, the through-beam switch is in a blocked state, and the PCB 3 sends a second signal to the outside.

Referring to FIG. 14 and FIG. 15, the brim portion 51 and the main shaft portion 52 define an annular semi-enclosed chamber (not indicated in the figure), the reset spring 6 has a first end and a second end that are away from each other, and the first end abuts against one side surface of the PCB 3 that is away from the through-beam switch, the second end is located in the semi-enclosed chamber, and the second end abuts against the pressing member 8 to provide a force for the pressing member 8 towards the first position.

In some examples, the detection mechanism may be disposed on the actuation box top plate 2; or, in some other examples, the detection mechanism may be disposed on the instrument box bottom plate 5; further, in some other examples, the detection mechanism may be disposed on both the actuation box top plate 2 and the instrument box bottom plate 5, so as to achieve the purpose of monitoring the coupling states between the actuation box top plate 2 and the sterile isolation plate 20 and the instrument box bottom plate 5.

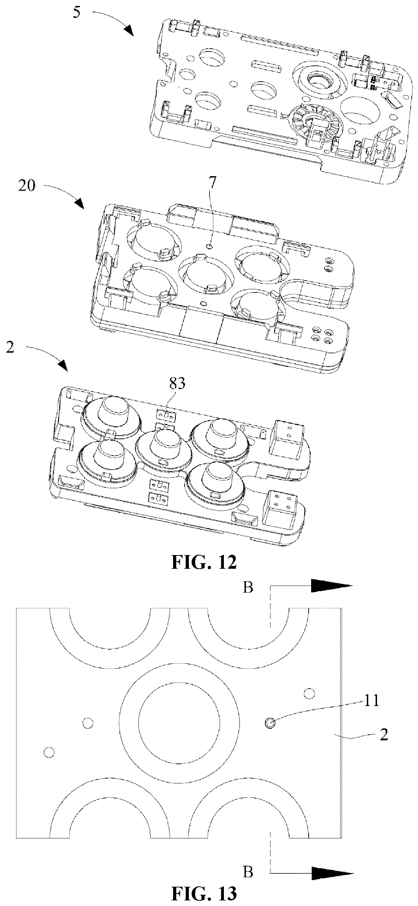

In some examples of the embodiments of the present disclosure, the detection mechanism is disposed on the actuation box top plate 1, which is taken as an example for illustration. Referring to FIG. 13 to FIG. 15, the actuation box top plate 2 is provided with a groove 11 at the bottom thereof, a plurality of second through holes 12 at the top thereof, and a plurality of third through holes 13 between the groove 11 and the second through holes 12.

Numbers of the second through holes 12 and the third through holes 13 are the same as the number of the pressing devices, and one pressing device is correspondingly arranged in one third through hole 13. The second through holes 12, the third through hole 13 and the groove 11 are communicated in sequence to penetrate through the actuation box top plate 2 along an axial direction.

The PCB 3 is fixedly arranged at the bottom of the actuation box top plate 2, and the PCB 3 and the through-beam switch are both received in the groove 11 to reduce an overall space of the two. The second through hole 12 is matched with the pressing boss 83 of the pressing member 8, forming a shaft-hole matching structure, and when the pressing member 8 is in the first position, at least a part of the pressing boss 83 protrudes axially relative to the upper surface of the actuation box top plate 2. The third through hole 13 is matched with the brim portion 82 of the pressing member 8, forming a shaft-hole matching structure, so as to limit the movement of the pressing member 8 along a vertical direction.

In some examples, taking the coupling of the actuation box top plate 2 and the sterile isolation plate 20 as an example, and in combination with FIG. 13 to FIG. 15, when the actuation box top plate 2 and the sterile isolation plate 20 are not coupled, the pressing member 8 is maintained in the first position (for example, as shown in FIG. 14) under the action of the reset spring 6, the through-beam switch is in an activated state, and the PCB 3 sends a first signal to the outside; when the sterile isolation plate 20 is coupled to the actuation box top plate 2, the pressing member 8 is pressed down to the second position by the sterile isolation plate 20 (for example, as shown in FIG. 15), the through-beam switch is in a blocked state, and the PCB 3 sends a second signal to the outside. Since the stroke of the pressing member 8 is short, if the sterile isolation plate 20 is not coupled to the actuation box top plate 2 or the two are not firmly coupled, the pressing member 8 is not pressed down and is in the first position. Therefore, the coupling state between the sterile isolation plate 20 and the actuation box top plate 2 may be determined through the signals sent by the PCB 3. In addition, the pressing device adopts a through-beam switch, which is sensitive in response and occupies a small space, and can further improve the reliability of the detection mechanism and simplify its internal structure.

In some examples, referring to FIG. 2 and FIG. 16 to FIG. 17, the detection mechanism provided by some embodiments of the present disclosure is configured with a pair of first pressing devices away from each other and a pair of second pressing devices away from each other, and a pair of axially movable transmission rods 7 are disposed on the sterile isolation plate 20. The pair of transmission rods 7 correspond to the pair of second pressing devices and can contact the pressing members 8 of the second pressing devices.

When the sterile isolation plate 20 is coupled on the actuation box top plate 2, the pressing member 8 of each of the plurality of first pressing devices is limited to the first position; when the actuation box top plate 2, the sterile isolation plate 20 and the instrument box bottom plate 5 are coupled in sequence, the pressing members 8 of the pair of second pressing devices contact the pair of transmission rods 7 and are limited to the second position. In this way, the overall volume required for the detection mechanism can be reduced, and it may avoid a situation in which one sides of the corresponding coupling components are not firmly coupled is missed. It can be understood that in other embodiments, the PCB 3 may be omitted, and a plurality of pressing devices are separately set on the actuation box top plate or/and the instrument box bottom plate to monitor the coupling states between the actuation box top plate 2 and the sterile isolation plate 20, and between the sterile isolation plate 20 and the instrument box bottom plate 5.

The above illustrates and describes the basic principles, main features and advantages of the present disclosure. Those skilled in the art should understand that the present disclosure is not limited by the above embodiments, and the above embodiments and the descriptions are only for illustrating the principles of the present disclosure. Various changes and improvements may be made to the present disclosure without departing from the spirit and scope of the present disclosure, and the protection scope of the present disclosure is defined by the attached claims, description and their equivalents.

## Claims

1. A surgical robot, comprising:
an actuation box capable of carrying a power device that provides power, the actuation box comprising an actuation box top plate arranged at a top portion, and the actuation box top plate defining a central axis;
a sterile isolation plate, the sterile isolation plate being detachably coupled to an upper side of the actuation box top plate;
an instrument box, the instrument box comprising an instrument box bottom plate arranged at a bottom portion, and the instrument box bottom plate being detachably coupled to an upper side of the sterile isolation plate; and
a coupling monitoring mechanism arranged on the actuation box top plate, the coupling monitoring mechanism comprising at least one pair of first monitoring switches and at least one pair of second monitoring switches, wherein the at least one pair of first monitoring switches are exposed to outside relative to the actuation box top plate and are arranged at two sides of the central axis away from each other; the at least one pair of second monitoring switches are exposed to outside relative to the actuation box top plate and are arranged at two sides of the central axis away from each other;
the first monitoring switch is configured to send out a first trigger signal when the sterile isolation plate is coupled to the actuation box top plate, and the second monitoring switch is configured to send out a second trigger signal when the instrument box bottom plate is coupled to the sterile isolation plate.

2. The surgical robot according to claim 1, wherein the first monitoring switch comprises a first pressing rod protruding outward and a first trigger switch, and wherein the first pressing rod is retractable, the first trigger switch cooperates with the first pressing rod, and when the sterile isolation plate is coupled to the actuation box top plate, the first pressing rod is compressed and triggers the first trigger switch.

3. The surgical robot according to claim 1, wherein the second monitoring switch comprises a second pressing rod protruding outward and a second trigger switch, and wherein the second pressing rod is retractable, the second trigger switch cooperates with the second pressing rod, and when the instrument box bottom plate is coupled on the sterile isolation plate, the second pressing rod is compressed and triggers the second trigger switch.

4. The surgical robot according to claim 3, wherein a pair of transmission rods that are axially movable are arranged on the sterile isolation plate, and when the sterile isolation plate is coupled to the actuation box top plate, the pair of transmission rods correspondingly contact a pair of the second pressing rods to transmit pressure from the instrument box bottom plate.

5. The surgical robot according to claim 1, wherein the first monitoring switches and the second monitoring switches are both integrated on a circuit board; the actuation box top plate defines a groove exposed from a bottom thereof, and the circuit board is arranged in the groove; the actuation box top plate is formed with a metal protective layer on a top portion thereof to protect the circuit board.

6. The surgical robot according to claim 1, wherein either of the first monitoring switch and the second monitoring switch comprises a plurality of pressing devices arranged on the actuation box top plate, and wherein the pressing device comprise a through-beam switch, an axially movable pressing device and a reset spring, the through-beam switch comprising a light transmitter and a light receiver arranged opposite to each other, and the pressing device has a first position and a second position;
in the first position, at least a portion of the pressing member protrudes axially relative to the actuation box and is separated from the through-beam switch;
in the second position, the pressing member is completely received in the actuation box and is at least partially located between the light transmitter and the receiver; the reset spring abuts against the pressing member and provides a force for the pressing member towards the first position.

7. The surgical robot according to claim 6, wherein either of the first monitoring switch and the second monitoring switch further comprises: a PCB fixedly disposed in the actuation box top plate, wherein the PCB is provided thereon with a plurality of first through holes; the through-beam switch is fixedly disposed on the same side surface of the PCB, the through-beam switch is arranged corresponding to the first through hole, and the light transmitter and the light receiver are oppositely located at two sides of the first through hole.

8. The surgical robot according to claim 7, wherein the pressing member comprises a main shaft portion, and wherein the main shaft portion matches with the first through hole in a shaft-hole manner, and when the pressing member is located at the second position, a lower portion of the main shaft portion passes through the first through hole, and the main shaft portion extends between the light transmitter and the light receiver.

9. The surgical robot according to claim 8, wherein the pressing member further comprises a brim portion, wherein a diameter of the brim portion is larger than a diameter of the main shaft portion, an outer edge of the brim portion is configured to extend axially and form an annular semi-enclosed chamber with the main shaft portion; one end of the reset spring abuts against the PCB, and the other end is located in the semi-enclosed chamber.

10. The surgical robot according to claim 6, wherein the actuation box top plate is provided therein with a plurality of third through holes; the pressing member is correspondingly arranged at the third through holes; and the pressing member includes a brim portion, the brim portion is located in the third through hole, and the brim portion matches with the third through hole in a shaft-hole manner to limit a moving direction of the pressing member.

11. The surgical robot according to claim 6, wherein the actuation box top plate is provided thereon with a plurality of second through holes, the plurality of second through holes are exposed to the outside; the pressing member is correspondingly arranged at the second through hole, and the pressing member includes a pressing boss, the pressing boss matches with the second through hole in a shaft-hole manner, and when the pressing member is located at the first position, the pressing boss is exposed to the outside.

12. A detection mechanism for a surgical robot, the surgical robot comprising an actuation box having an actuation box top plate, a sterile isolation plate and an instrument box having an instrument box bottom plate, the instrument box being detachably coupled to the actuation box via the sterile isolation plate, wherein the detection mechanism comprises a plurality of pressing devices arranged on the actuation box top plate and/or the instrument box bottom plate, the pressing device comprising a through-beam switch, an axially movable press member and a reset spring, and wherein the through-beam switch comprise a light transmitter and a light receiver arranged opposite to each other; and the press member having a first position and a second position;
in the first position, at least a portion of the pressing member protrudes axially relative to the actuation box or the instrument box, and is separated from the through-beam switch;
in the second position, the pressing member is completely received in the actuation box or the instrument box, and at least a portion of the pressing member is located between the light transmitter and the receiver; the reset spring abuts against the pressing member and provides a force for the pressing member towards the first position.

13. The detection mechanism according to claim 12, wherein the detection mechanism further comprises a PCB, the PCB being provided with a plurality of first through holes and being fixedly arranged in the actuation box top plate or the instrument box bottom plate; the through-beam switch is arranged corresponding to the first through hole, the through-beam switch is fixedly arranged on the same side surface of the PCB, and the light transmitter and the light receiver are oppositely located at two sides of the first through hole.

14. The detection mechanism according to claim 13, wherein the pressing member comprises a main shaft portion, and wherein the main shaft portion matches with the first through hole in a shaft-hole manner, and when the pressing member is located at the second position, a lower portion of the main shaft portion passes through the first through hole and extends between the light transmitter and the light receiver.

15. The detection mechanism according to claim 14, wherein the pressing member further comprises a brim portion, and wherein a diameter of the brim portion is larger than a diameter of the main shaft portion; an outer edge of the brim portion is configured to extend axially and form an annular semi-enclosed chamber with the main shaft portion; one end of the reset spring abuts against the PCB, and the other end is located in the semi-enclosed chamber.

16. The detection mechanism according to claim 13, wherein a bottom of the actuation box top plate is recessed inward to form a groove, and the PCB is fixedly disposed in the actuation box top plate and is located in the groove.

17. The detection mechanism according to claim 13, wherein the light transmitter and the light receiver are both electrically connected to the PCB, and the light receiver is a photoresistor.

18. The detection mechanism according to claim 12, wherein the actuation box top plate or the instrument box bottom plate is provided therein with a plurality of third through holes; the pressing member is correspondingly arranged at the third through hole, and the pressing member includes a brim portion, the brim portion is located in the third through hole, and the brim portion matches with the third through hole in a shaft-hole manner to limit moving direction of the pressing member.

19. The detection mechanism according to claim 12, wherein the actuation box top plate or the instrument box bottom plate is provided with a plurality of second through holes, and the plurality of second through holes are exposed to the outside; the pressing member is correspondingly arranged at the second through hole, and the pressing member comprises a pressing boss, and the pressing boss matches with the second through hole in a shaft-hole manner, and when the pressing member is located at the first position, the pressing boss is exposed to the outside.

20. The detection mechanism according to claim 12, wherein the detection mechanism comprises a pair of first pressing devices away from each other; the first pressing devices are arranged on the actuation box top plate, and when the sterile isolation plate is coupled to the actuation box top plate, pressing members of the first pressing devices are in the second position.

21. The detection mechanism according to claim 20, wherein the detection mechanism further comprises a pair of second pressing devices away from each other; and a pair of axially movable transmission rods are arranged on the sterile isolation plate, when the actuation box top plate, the sterile isolation plate and the instrument box bottom plate are sequentially coupled, the pair of transmission rods respectively abut against pressing members of the pair of second pressing devices, and the pressing members of the pair of second pressing devices are both in the second position.

22. A detection method for the surgical robot according to any one of claims 1 to 11, comprising the following steps:
S1: collecting a first trigger signal, generating a first coupling success information indicating that the sterile isolation plate and the actuation box are successfully coupled if each of the first monitoring switches sends out the first trigger signal, and sending a ready-to-couple information to an outside to prepare for coupling the instrument box; otherwise, generating a first coupling failure information, and sending a first fault information to the outside;
S2: collecting a second trigger signal, and generating a second coupling success information indicating that the instrument box and the sterile isolation board are successfully coupled if each of the second monitoring switches sends out the second trigger signal; otherwise, sending out a second fault information.

23. The detection method according to claim 22, wherein the first fault information comprises at least one of: an indicator light of the actuation box flashes or changes color, a voice prompt indicating that the isolation board is incorrectly coupled, and a prompt shown on a doctor end-display indicating that the sterile isolation board is incorrectly coupled;
and/or,
the second fault information comprises at least one of: an indicator light of the actuation box flashes or changes color, a voice prompt indicating that the instrument box is incorrectly coupled, and a prompt shown on a doctor end-display indicating that the instrument box is incorrectly coupled.

24. A detection method for the surgical robot according to any one of claims 1 to 11, comprising:
collecting a first trigger signal, and confirming that the sterile isolation plate is successfully coupled to the actuation box in response to first trigger signals sent by each of the at least one pair of first monitoring switches;
sending a ready-to-couple information to be ready to couple the instrument box;
collecting a second trigger signal, and confirming that the instrument box and the sterile isolation board are successfully coupled in response to second trigger signals sent by each of the at least one pair of second monitoring switches.

25. The detection method according to claim 24, wherein the detection method further comprises:
confirming that the sterile isolation plate fails to be coupled to the actuation box in response to any one of the at least one pair of first monitoring switches not sending the first trigger signal;
sending a first fault information;
confirming that the instrument box fails to be coupled to the sterile isolation board in response to any one of the at least one pair of second monitoring switches not sending the second trigger signal; and
sending a second fault information.

26. The detection method according to claim 25, wherein the first fault information or the second fault information comprises at least one of: an indicator light of the actuation box flashes, an indicator light of the actuation box changes color, a voice prompt, and a prompt shown on a display.
